(19) Europäisches Patentamt
European Patent Office
Office européen des brevets

(11) **EP 2 327 147 B1**

(12) **EUROPÄISCHE PATENTSCHRIFT**

(45) Veröffentlichungstag und Bekanntmachung des Hinweises auf die Patenterteilung:
**26.09.2018 Patentblatt 2018/39**

(21) Anmeldenummer: **09782184.7**

(22) Anmeldetag: **26.08.2009**

(51) Int Cl.:
**H02N 15/00** *(2006.01)*    **A61B 34/00** *(2016.01)*

(86) Internationale Anmeldenummer:
**PCT/EP2009/060957**

(87) Internationale Veröffentlichungsnummer:
**WO 2010/034582 (01.04.2010 Gazette 2010/13)**

(54) **SPULENSYSTEM ZUR BERÜHRUNGSLOSEN MAGNETISCHEN NAVIGATION EINES MAGNETISCHEN KÖRPERS IN EINEM ARBEITSRAUM**

COIL SYSTEM FOR TOUCHLESS MAGNETIC NAVIGATION OF A MAGNETIC BODY IN A WORKING SPACE

SYSTEME DE BOBINES POUR NAVIGATION MAGNETIQUE D'UN OBJET MAGNETIQUE DANS UN ESPACE DE TRAVAIL SANS TOUCHER LEDIT OBJET

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **26.09.2008 DE 102008049198**

(43) Veröffentlichungstag der Anmeldung:
**01.06.2011 Patentblatt 2011/22**

(73) Patentinhaber: **Siemens Healthcare GmbH 91052 Erlangen (DE)**

(72) Erfinder:
• **JULOSKI, Aleksandar 90491 Nuernberg (DE)**
• **REINSCHKE, Johannes 90425 Nürnberg (DE)**

(56) Entgegenhaltungen:
WO-A2-2004/030198    DE-A1- 10 341 092
US-A- 4 585 282    US-A1- 2005 062 562

• **BERRY M V ET AL: "OF FLYING FROGS AND LEVITRONS" EUROPEAN JOURNAL OF PHYSICS, INSTITUTE OF PHYSICS PUBLISHING, BRISTOL, GB, Bd. 18, Nr. 4, 1. Juli 1997 (1997-07-01), Seiten 307-313, XP000700340 ISSN: 0143-0807**
• **Dr Med Wolfgang ET AL: "Internistische Gemeinschaftspraxis", , 16 August 2017 (2017-08-16), XP55399177, Retrieved from the Internet: URL:http://www.gastro-muc-solln.de/pdf/auf klaerung-kapsel-endoskopie.pdf [retrieved on 2017-08-17]**

**Beschreibung**

Die Erfindung betrifft ein Spulensystem zur berührungslosen magnetischen Navigation eines magnetischen Körpers in einem Arbeitsraum.

Spulensysteme zur berührungslosen magnetischen Navigation eines magnetischen Körpers erzeugen mit einer Mehrzahl von Spulen ein Magnetfeld, welches mit dem magnetischen Körper wechselwirkt, wodurch magnetische Kräfte und Drehmomente generiert werden, welche die Bewegung des magnetischen Körpers verursachen. Über die entsprechenden Ströme in den einzelnen Spulen des Spulensystems kann dabei die magnetische Kraft und das magnetische Drehmoment, welche auf den magnetischen Körper wirken, geeignet eingestellt werden.

Insbesondere finden Spulensysteme der obigen Art im medizinischen Bereich Verwendung. Dabei wird mit dem magnetischen Körper ein Patient im Arbeitsraum des Spulensystems untersucht. Der Arbeitsraum ist dabei von außen zugänglich und in diesem Raum haben die magnetischen Kräfte des Spulensystems eine ausreichende Wirkung auf den magnetischen Körper. Zur Durchführung der Untersuchung werden der magnetische Körper, der sich im Patienten befindet, und der zu untersuchende Teil des Patientenkörpers in den Arbeitsraum des Spulensystems eingebracht. Der magnetische Körper stellt dabei eine Sonde dar, mit der Messungen an - insbesondere Bildaufnahmen von - inneren Organen des Patienten gemacht werden können.

Ein Spulensystem mit magnetischer Sonde wird beispielsweise in der Gastroenterologie, insbesondere der Gastroskopie eingesetzt, siehe WO 2007/077922 A1. Während der endoskopischen Untersuchung wird der Magen des Patienten teilweise mit Wasser gefüllt und der Patient schluckt eine entsprechende Sonde, welche einen Permanentmagneten und eine Kamera enthält.

Der Magen des Patienten befindet sich dabei im Arbeitsraum des Spulensystems bzw. wird nach Schlucken der Sonde in den Arbeitsraum eingebracht. Unter Verwendung der durch das Spulensystem erzeugten magnetischen Kräfte und Drehmomente wird die Sonde so bewegt, dass Aufnahmen von den zu untersuchenden Arealen der Patientenmagenschleimhaut entstehen. Dabei ist es notwendig, dass durch geeignete Bestromung der Spulen ein inhomogenes Magnetfeld dergestalt erzeugt werden kann, dass durch die Wechselwirkung dieses Magnetfeldes mit dem Permanentmagneten in der Sonde die Sonde geeignet positioniert und in dieser Position gehalten wird.

Aus dem Stand der Technik sind verschiedene Ansätze bekannt, um einen magnetischen Körper relativ zu einem Spulensystem geeignet zu positionieren. Aus dem Dokument WO 2006/014011 A1 ist ein Spulensystem bekannt, bei dem der zu untersuchende Patient mechanisch in Bezug auf das Spulensystem während der Untersuchung bewegt wird. Das Spulensystem ist derart konstruiert, dass ein einzelner Raumpunkt existiert, der fest in Bezug auf das Spulensystem ist. Wirken auf den magnetischen Körper keine externen Kräfte, bewegt sich der Körper aufgrund der ausgeübten magnetischen Kräfte und Drehmomente hin zu diesem Raumpunkt. Wenn der magnetische Körper diesen Raumpunkt erreicht hat, bleibt er an diesem Raumpunkt, sofern von außen keine Kräfte ausgeübt werden. Es erweist sich hierbei als nachteilhaft, dass zur Bewegung des magnetischen Körpers entweder das Spulensystem oder der Patient oder beiden mechanisch bewegt werden müssen. Dies ist insbesondere bei Anwendungen problematisch, bei denen eine schnelle Bewegung des magnetischen Körpers hin zu einer vorgegebenen Position erforderlich ist.

Aus dem Stand der Technik sind ferner Systeme bekannt, bei denen das Spulensystem durch eine oder mehrere Permanentmagnete zur Bewegung eines magnetischen Körpers ersetzt wird, siehe z.B. US 7,019,610 B2. Auch in diesen Systemen wird die Bewegung hin zu einer vorgegebenen Position durch eine mechanische Bewegung des Patienten bzw. des Permanentmagneten bzw. von Patient und Permanentmagnet erreicht.

Es sind ferner Spulensysteme bekannt, bei denen ohne mechanische Bewegung durch entsprechende Einstellung der magnetischen Felder und Feldgradienten an der Position des magnetischen Körpers eine nur durch die Ströme in dem Spulensystem verursachte Bewegung des magnetischen Körpers erreicht wird (siehe z.B. WO 2006/092421 A1). Dabei ist es jedoch erforderlich, dass die Position und Orientierung des magnetischen Körpers bekannt ist, was wiederum erfordert, dass die Position des magnetischen Körpers zusätzlich gemessen werden muss.

Eine Anlage zur berührungsfreien Bewegung oder Fixierung eines magnetischen Körpers wird ferner in DE 103 41 092 A1 beschrieben. Die Anlage umfasst Detektionsmittel zur Erfassung einer Ist-Position und Spulen zur Erzeugung eines Magnetfelds. Das Magnetfeld wird so eingestellt, dass eine Abweichung einer Soll-Position von der gemessenen Ist-Position minimiert wird.

Aufgabe der Erfindung ist es, die oben beschriebenen Nachteile des Standes der Technik zu umgehen und ein Spulensystem zu schaffen, mit denen auf einfache Weise ein magnetischer Körper berührungslos navigiert werden kann.

Diese Aufgabe wird durch das Spulensystem gemäß Patentanspruch 1 gelöst. Weiterbildungen der Erfindung sind in den abhängigen Ansprüchen definiert.

Das erfindungsgemäße Spulensystem umfasst eine Mehrzahl von Spulen und eine Stromsteuerung zur Steuerung der jeweiligen Ströme in der Mehrzahl von Spulen. Die Stromsteuerung ist dabei derart ausgestaltet, dass zur Navigation eines magnetischen Körpers an eine variabel vorgebbare Position in dem Arbeitsraum des Spulensystems die Ströme in der Mehrzahl von Spulen derart eingestellt werden, dass die durch die Ströme generierten magnetischen Kräfte, welche auf den magnetischen Körper an einer Mehrzahl von Positionen am Rand einer konvexen Umgebung um die vorgebbare Position wirken, in die Umgebung hinein weisen. "Variabel vorgebbar" bedeutet dabei, dass die Position im Arbeitsraum nicht fix ist, sondern durch die Stromsteuerung verschieden eingestellt werden kann. Der magnetische Körper ist insbesondere derart ausgestaltet, dass er ein vorgegebenes magnetisches Dipolmoment in eine vorbestimmte Richtung aufweist.

Erfindungsgemäß sind die zur Navigation des magnetischen Körpers an die vorgebbare Position einzustellenden Ströme die Lösung eines Optimierungsproblems mit der Randbedingung, dass die durch die Ströme generierten magnetischen Kräfte, welche auf den magnetischen Körper an mehreren Positionen am Rand der konvexen Umgebung um die vorgebbare Position wirken, in die Umgebung hinein weisen, wobei die Stromsteuerung eine Bewegung des magnetischen Körpers mit zwei translatorischen Freiheitsgraden oder weniger berücksichtigt.

Das Optimierungsproblem stellt in einer bevorzugten Ausführungsform ein lineares Programm bzw. ein quadratisches Programm dar, welche mit bekannten Standard-Optimierungsverfahren zuverlässig und in kurzer Rechenzeit gelöst werden können.

Durch eine derartige Steuerung der Ströme wird sichergestellt, dass unabhängig von der aktuellen Position des magnetischen Körpers im Arbeitsraum immer eine Bewegung des Körpers hin zu der vorgebbaren Position gewährleistet ist. Die vorgebbare Position kann dabei beispielsweise über eine Bedienerschnittstelle durch den Bediener eingestellt werden. Unter konvexer Umgebung wird erfindungsgemäß eine Umgebung verstanden, welche keine nach innen gewölbten Bereiche aufweist. Die konvexe Umgebung muss dabei im Arbeitsraum des Spulensystems liegen. Vorzugsweise liegt die Umgebung dabei in der Nachbarschaft zu der vorgebbaren Position. Nachbarschaft bedeutet insbesondere, dass der maximale Abstand des Rands des Umgebung 10% oder weniger von der größten Ausdehnung des Spulensystems ist. Für medizinische Anwendungen haben sich insbesondere Umgebungen als praktikabel erwiesen, deren Rand einen maximalen Abstand zwischen 0,005 m und 0,1 m, vorzugsweise von 0,01 m, zu der vorgebbaren Position hat.

Das erfindungsgemäße Spulensystem hat den Vorteil, dass nur durch eine entsprechende Stromsteuerung ein Magnetfeld-Maximum ("Peak") mit im Wesentlichen auf die vorgebbare Position weisenden Kräfte geschaffen werden kann. Dabei ist es nicht erforderlich, dass das Spulensystem mechanisch bewegt wird und die aktuelle Position des magnetischen Körpers im Arbeitsraum bekannt ist. Es kann somit auf eine Positionsbestimmung dieser aktuellen Position verzichtet werden.

In einer bevorzugten Ausführungsform ist die Umgebung um die vorgebbare Position ein Polyeder und/oder ein Polygon, z.B. ein Dreieck, Viereck oder ein anderes Vieleck. Ein Polygon kommt insbesondere dann in Betracht, wenn die Bewegung des magnetischen Körpers weniger als drei translatorische Freiheitsgrade aufweist. Vorzugsweise liegen die mehreren Positionen, auf welche die magnetischen Kräfte am Rand der Umgebung für einen dort positionierten magnetischen Körper wirken, an einem oder mehreren der Eckpunkte des Polygons und/oder Polyeders, insbesondere an allen Eckpunkten des Polygons und/oder Polyeders.

In einer bevorzugten Ausführungsform des Spulensystems ist die Stromsteuerung derart ausgestaltet, dass die Ströme im Betrieb des Spulensystems für die vorgebbare Position berechnet werden. Es werden somit in Realzeit für eine bestimmte Position die geeigneten Ströme ermittelt. Ebenso ist es möglich, dass die einzustellenden Ströme für eine Vielzahl von vorgebbaren Positionen in einem Speicher der Stromsteuerung hinterlegt sind. Wird eine Raumposition, zu der sich der magnetische Körper bewegen soll, beispielsweise über eine Bedienerschnittstelle festgelegt, werden die entsprechenden einzustellenden Ströme dann aus diesem Speicher ausgelesen.

In einer bevorzugten Ausführungsform der Erfindung ist das Optimierungsproblem die Minimierung des Betrags bzw. der 2-Norm (d.h. der euklidischen Norm) des Vektors der Ströme in der Mehrzahl von Spulen unter Berücksichtigung der weiteren Randbedingung, dass die durch die Ströme generierten magnetischen Kräfte, welche auf den magnetischen Körper an den mehreren Positionen am Rand der konvexen Umgebung um die vorgebbare Position wirken, betragsmäßig jeweils einen vorbestimmten Wert überschreiten.

In einer weiteren Ausgestaltung des erfindungsgemäßen Spulensystems werden bei der Lösung des Optimierungsproblems die Ströme unterschiedlich gewichtet. Statt der 2-Norm des Vektors der Spulenströme wird dabei zum Beispiel die 2-Norm eines gewichteten Vektors der Spulenströme minimiert werden, wobei jeder einzelne Spulenstrom mit der Quadratwurzel des Ohmschen Widerstandes der Spule gewichtet wird. Dadurch wird die Ohmsche Gesamtverlustleistung in der Mehrzahl der Spulen minimiert.

Alternativ oder zusätzlich kann das Optimierungsproblem auch derart definiert sein, dass die durch die Ströme generierten magnetischen Kräfte, welche auf den magnetischen Körper an den mehreren Positionen am Rand der konvexen Umgebung um die vorgebbare Position wirken, maximiert werden. Dabei wird als weitere Randbedingung berücksichtigt, dass die Ströme der Mehrzahl von Spulen betragsmäßig unterhalb eines jeweiligen Maximalwerts liegen.

In einer besonders bevorzugten Ausführungsform wird bei der Lösung der oben beschriebenen Optimierungsprobleme als zusätzliche Randbedingung berücksichtigt, dass das an der vorgebbaren Position durch die Ströme erzeugte Magnetfeld im Wesentlichen in eine vorgegebene Richtung weist und einen Betrag hat, der einen vorgegebenen Wert überschreitet. Auf diese Weise wird sichergestellt, dass der magnetische Körper auch eine vorbestimmte Orientierung an der vorgebbaren Position hat.

Das erfindungsgemäße Spulensystem wird vorzugsweise in einem medizinischen Gerät eingesetzt, welches derart ausgestaltet ist, dass ein Patient in das Spulensystem positioniert werden kann und durch die Stromsteuerung ein magnetischer Körper in der Form einer Sonde zur Untersuchung der Organe des Patienten an vorgebbaren Positionen innerhalb des Patientenkörpers navigiert werden kann.

Mit Hilfe eines erfindungsgemäßen Spulensystems kann ein Verfahren zur berührungslosen magnetischen Navigation eines magnetischen Körpers in einem Arbeitsraum durchgeführt werden. Dabei werden zur Navigation des magnetischen Körpers an eine vorgebbare Position in dem Arbeitsraum die Ströme in der Mehrzahl von Spulen des Spulensystems durch die Stromsteuerung des Spulensystems erfindungsgemäß derart eingestellt, dass die durch die Ströme generierten magnetischen Kräfte, welche auf den magnetischen Körper an mehreren Positionen am Rand einer konvexen Umgebung um die vorgebbare Position wirken, in die Umgebung hinein weisen, wobei die Stromsteuerung eine Bewegung des magnetischen Körpers mit zwei translatorischen Freiheitsgraden oder weniger berücksichtigt. Erfindungsgemäß sind die zur Navigation des magnetischen Körpers an die vorgebbare Position einzustellenden Ströme die Lösung eines Optimierungsproblems mit der Randbedingung, dass die durch die Ströme generierten magnetischen Kräfte, welche auf den magnetischen Körper an mehreren Positionen am Rand der konvexen Umgebung um die vorgebbare Position wirken, in die Umgebung hinein weisen.

Ausführungsbeispiele der Erfindung werden nachfolgend anhand der beigefügten Figuren detailliert beschrieben.

Es zeigen:

Fig. 1      eine schematische Darstellung eines magnetischen Körpers in der Form einer Kapsel für medizinische Anwendungen;

Fig. 2      eine schematische Darstellung der Konfiguration der magnetischen Kräfte zur Bewegung einer Kapsel an eine Raumposition gemäß einer Ausführungsform der Erfindung;

Fig. 3      eine schematische Darstellung des Optimierungsproblems der Erfindung für die Bewegung einer Kapsel mit zwei translatorischen Freiheitsgraden.

Nachfolgend wird die Erfindung anhand eines medizinischen Geräts zur endoskopischen Untersuchung von Organen eines Patienten erläutert. Das medizinische Gerät umfasst ein Spulensystem, welches insbesondere analog zu dem Spulensystem der Druckschrift WO 2006/092421 A1 aufgebaut ist. Dieses Spulensystem umfasst 14 einzeln ansteuerbare Spulen zur Erzeugung entsprechender Magnetfelder. Die einzelnen Spulen sind dabei derart ausgestaltet, dass das magnetische Feld bzw. der Feldgradient der jeweiligen Spulen sich nicht auf einen oder mehrere Raumpositionen in dem durch die Spulen vorgegebenen Arbeitsraum konzentriert. Gegebenenfalls können auch andere Spulensysteme eingesetzt werden, wobei als Minimum acht Spulen verwendet werden sollten, um die magnetischen Freiheitsgrade unabhängig voneinander steuern zu können. Trotz des gleichen Aufbaus wie das Spulensystem der Druckschrift WO 2006/092421 A1 unterscheidet sich das in der nachfolgend beschriebenen Ausführungsform verwendete Spulensystem in der Steuerung der Ströme der einzelnen Spulen.

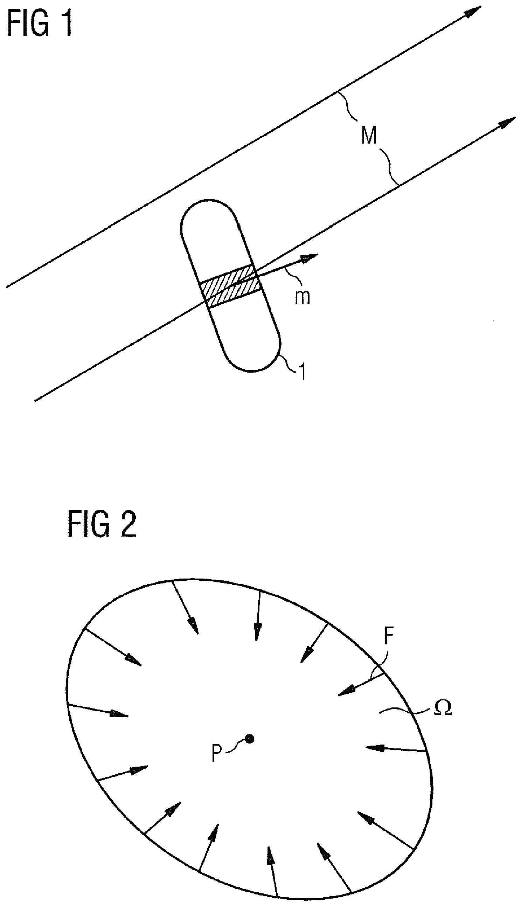

In der hier beschriebenen Ausführungsform wird durch das Spulensystem eine magnetische Kapsel 1 bewegt, welche in Fig. 1 schematisch wiedergegeben ist. Die Kapsel stellt dabei eine endoskopische Sonde dar, welche von dem Patienten geschluckt wird, um entsprechende Untersuchungen im Magen-Darmtrakt des Patienten vorzunehmen. Die Kapsel ist ein magnetischer Körper mit einem magnetischen Dipolmoment m. Beim Anlegen eines Magnetfelds, welches in Fig. 1 schematisch durch die Feldlinien M angedeutet ist, wird ein Drehmoment auf die Kapsel 1 erzeugt, welches das magnetische Dipolmoment der Kapsel in Richtung des Magnetfelds ausrichtet.

Die Kapsel 1 enthält eine (nicht gezeigte) Kamera und wird von dem Patienten vor der endoskopischen Untersuchung geschluckt. Der Patient ist dabei oder wird danach im Arbeitsraum des Spulensystems positioniert. Die Kapsel kann dann durch entsprechende Einstellung der Ströme in dem Spulensystem bewegt werden und insbesondere auch in eine gewünschte Orientierungsrichtung ausgerichtet werden. Mit der Kapsel können beispielsweise gastroenterologische Untersuchungen gemacht werden. Dabei trinkt der Patient vor und gegebenenfalls auch während der Untersuchung eine ausreichende Menge Wasser, so dass bei der Untersuchung die Kapsel im Wasser oder an der Wasseroberfläche bewegt wird. Durch geeignete Einstellung der Ströme in den Spulen kann dann die Kapsel an der Wasseroberfläche zu den zu untersuchenden Bereichen im Magen bewegt und orientiert werden, und auch Nahaufnahmen, bei denen sich die Kapsel gänzlich im Wasser unterhalb der Wasseroberfläche befindet, sind möglich. Die Kapsel verfügt dabei über einen Hochfrequenz-Sender, mit dem die aufgenommenen Bilder ausgesendet werden und mit einem entsprechenden Empfänger außerhalb des Patienten empfangen werden. Dieser Empfänger ist beispielsweise in einem Gürtel integriert, den der Patient bei der Untersuchung trägt.

In der nachfolgend beschriebenen Ausführungsform wird die Bewegung der Kapsel in einem Raum mit fünf Freiheitsgraden umfassend zwei translatorische und alle drei rotatorischen Freiheitsgrade betrachtet. Dies entspricht beispielsweise der oben beschriebenen Bewegung der Kapsel auf der Wasseroberfläche im Magen eines Patienten mit drei rotatorischen und lediglich zwei translatorischen Freiheitsgraden. Ziel der nachfolgend beschriebenen Steuerung der Ströme in den Spulen ist es nunmehr, in dem Arbeitsraum des Spulensystems die Ströme derart einzustellen, dass durch die Spulen ein Magnetfeld-Maximum an einer vorgegebenen Raumposition erzeugt wird, so dass sich die Kapsel hin zu dieser Raumposition bewegt und dort verbleibt. Die Raumposition kann dabei von einem Bediener, d.h. dem medizinischen Personal, über eine Bedienerschnittstelle geeignet eingestellt und verändert werden, um entsprechende Untersuchungen an den relevanten Organen des Patienten vorzunehmen.

Im Folgenden wird der dreidimensionale magnetische Dipolmoment-Vektor der Kapsel 1 mit $\vec{m}$ bezeichnet und es wird ein Spulensystem mit $n_{coils}$ Spulen betrachtet. Das magnetische Dipolmoment der Kapsel wird dabei durch ein geeignetes magnetisches Element innerhalb der Kapsel erzeugt. Darüber hinaus wird die spezifische dreidimensionale Raumposition, zu der sich die Kapsel hinbewegen soll, mit $P$ bezeichnet. Das durch den Stromfluss in den Spulen generierte Magnetfeld wird durch den dreidimensionalen Magnetfeldvektor $\vec{B}(P)$ repräsentiert. Die durch das Magnetfeld erzeugte Kraft in Abhängigkeit von dem magnetischen Dipolmoment der Kapsel und der Raumposition $P$ wird durch den dreidimensionalen Kraftvektor $\vec{F}(P,\vec{m})$ wiedergegeben. Die Ströme in den einzelnen Spulen werden durch einen Vektor $I$ mit $n_{coils}$ Einträgen repräsentiert, wobei jeder Eintrag den Stromfluss in einer Einzelspule wiedergibt. Es besteht dabei folgender Zusammenhang zwischen dem Stromvektor $I$ und dem daraus generierten Magnetfeld und der daraus generierten Kraft an der Raumposition $P$ :

$$\left[ \begin{array}{c} \vec{B} \\ \vec{F} \end{array} \right] = A(P,\vec{m})I$$

Dabei ist $A(P,\vec{m})$ eine $6 \times n_{coils}$-Matrix. Die Matrix $A(P,\vec{m})$ ist das Produkt aus zwei Matrizen $U(\vec{m})$ und $V(P)$, d.h. es gilt:

$$A(P,\vec{m}) = U(\vec{m})V(P)$$

Die Matrix $V(P)$ ist eine $8 \times n_{coils}$ Matrix, welche nicht nur von der spezifischen Raumposition P, sondern auch von der Geometrie des Spulensystems abhängt. Die Matrix ist dabei vorgegeben bzw. kann problemlos aus der spezifischen Geometrie des Spulensystems nach dem Gesetz von Biot-Savart für beliebige Raumpositionen bestimmt werden. Die weitere Matrix $U(\vec{m})$ ist eine 6x8 Matrix, welche wie folgt lautet:

$$U(\vec{m}) = \left[ \begin{array}{cccccccc} 1 & 0 & 0 & 0 & 0 & 0 & 0 & 0 \\ 0 & 1 & 0 & 0 & 0 & 0 & 0 & 0 \\ 0 & 0 & 1 & 0 & 0 & 0 & 0 & 0 \\ 0 & 0 & 0 & m_x & m_y & m_z & 0 & 0 \\ 0 & 0 & 0 & 0 & m_x & 0 & m_z & m_y \\ 0 & 0 & 0 & -m_z & 0 & m_x & m_y & -m_z \end{array} \right]$$

Dabei repräsentieren $m_x$, $m_y$, $m_z$ die x-, y- und z-Komponenten des magnetischen Dipolmoment-Vektors $\vec{m}$.

Zur Berechnung der Matrix $U(\vec{m})$ muss das magnetische Dipolmoment $\vec{m}$, d.h. die Orientierung der Kapsel, bekannt sein. Wie bereits dargelegt, wird ein Szenario betrachtet, bei dem sich die Kapsel im Raum mit fünf Freiheitsgraden umfassend alle drei rotatorischen Freiheitsgrade bewegen kann. Dies hat zur Folge, dass sich das magnetische Dipolmoment der Kapsel im Wesentlichen (d.h. mit vernachlässigbarem Fehler) in Richtung des auf die Kapsel wirkenden magnetischen Feldes $\vec{B}$ des Spulensystems ausrichtet. Um dies sicherzustellen, wird im Folgenden als Randbedingung vorgegeben, dass der Betrag des Magnetfelds größer als ein Minimalwert ist, d.h. es gilt:

$$\|\vec{B}\| \geq B_{min}$$

Dabei ist $B_{min}$ ein geeignet gewählter skalarer Wert, der so groß gewählt wird, dass das größtmögliche Stör-Drehmoment, das auf die Kapsel wirken kann, geteilt durch das Produkt aus $B_{min}$ und den Betrag von $\vec{m}$, kleiner ist als der Sinus des maximal zulässigen Raumwinkels zwischen $\vec{B}$ und $\vec{m}$.

Das magnetische Dipolmoment $\vec{m}$ der Kapsel ist bekannt. Somit kann die Beziehung zwischen dem Magnetfeld bzw. der ausgeübten Kraft und dem Stromvektor an der spezifischen Raumposition P wie folgt geschrieben werden:

$$\left[ \begin{array}{c} \vec{B} \\ \vec{F} \end{array} \right] = A(P)I$$

Wie bereits dargelegt, sollen die Ströme in dem Spulensystem nunmehr derart eingestellt werden, dass an dem Punkt P ein Magnetfeld mit einem Maximum erzeugt wird. Dies geschieht durch die Lösung eines konvexen Optimierungsproblems unter Berücksichtigung der Randbedingung, dass sich die magnetischen Kräfte in Bezug auf die spezifische Raumposition so ausrichten, wie in Fig. 2 angedeutet ist. Fig. 2 zeigt dabei die spezifische Raumposition $P$ sowie eine vorgegebene Umgebung $\Omega$ um die Raumposition. Aus Übersichtlichkeitsgründen ist dabei eine zweidimensionale Umgebung wiedergegeben, die Optimierung kann jedoch gegebenenfalls auch auf dreidimensionale Umgebungen angewendet werden. Die Ausrichtung der Kräfte an dem geschlossenen Rand dieser Umgebung ist dabei mit einer Vielzahl von Pfeilen angedeutet, wobei beispielhaft einer der Pfeile mit F bezeichnet ist. Die Ausrichtung der Kräfte muss dabei an den entsprechenden Positionen am Rand der Umgebung (welche den jeweiligen Ursprüngen der Pfeile entsprechen) derart sein, dass jede Kraft, die auf den magnetischen Körper an der jeweiligen Randposition ausgeübt wird, in die Umgebung $\Omega$ hinein weist. Dieses Kriterium wird in den nachfolgend erläuterten Optimierungsproblemen als notwendige Randbedingung berücksichtigt.

Die nachfolgend formulierten Optimierungsprobleme sind konvexe Optimierungsprobleme, welche verlässlich und effizient unter der Verwendung von hinlänglich aus dem Stand der Technik bekannten konvexen Optimierungsmethoden gelöst werden können. In dem hier beschriebenen Szenario der magnetischen Navigation einer magnetischen Kapsel kann das Optimierungsproblem auch in Echtzeit gelöst werden. Das heißt, die Stromsteuerung des Spulensystems enthält eine Recheneinheit, welche abhängig von der gewählten Orientierung und Position, zu der sich die Kapsel bewegen soll, ein entsprechendes Optimierungsproblem löst und die sich daraus ergebenden Stromwerte einstellt. Gegebenenfalls ist es auch möglich, für eine Vielzahl von Orientierungen und Positionen im Arbeitsraum des Spulensystems die entsprechend einzustellenden Ströme vorab zu ermitteln und in einem Speicher in der Stromsteuerung des Spulensystems zu hinterlegen.

Das erfindungsgemäß zu lösende Optimierungsproblem wird nachfolgend basierend auf einer Bewegung der Kapsel erläutert, bei der in y-Richtung keine Kräfte auf die Kapsel wirken und die Kapsel auch keine translatorische Bewegung in y-Richtung erfahren kann. Dies entspricht der obigen Bewegung mit zwei translatorischen Freiheitsgraden, wobei nur eine Translationsbewegung der Kapsel in die x- und z-Richtung möglich ist. Zusätzlich kann die Kapsel beliebig rotieren, d.h. für die Rotation existieren alle drei möglichen Freiheitsgrade.

Der Zusammenhang zwischen dem Stromvektor und dem Magnetfeld sowie der ausgeübten magnetischen Kraft kann wie folgt geschrieben werden:

$$B_x(P) = A_1(P)I$$

$$B_y(P) = A_2(P)I$$

$$B_z(P) = A_3(P)I$$

$$F_x(P) = A_4(P)I$$

$$F_y(P) = A_5(P)I$$

$$F_z(P) = A_6(P)I$$

Dabei bezeichnet $A_i$ die Zeile i der obigen Matrix $A(P)$.

Die gewünschte Orientierung $\vec{m}_{desired}$ des magnetischen Dipols und somit die gewünschte Orientierung des magnetischen Feldes wird vorab geeignet fest gewählt und ist in dem hier beschriebenen Ausführungsbeispiel beliebig. Es besteht immer die Möglichkeit, das (x, y, z)-Koordinatensystem derart auszurichten, dass der Vektor $\vec{m}_{desired}$ in die x-Achse des Koordinatensystems ausgerichtet ist. Somit wird ohne Beschränkung der Allgemeinheit nachfolgend das Szenario betrachtet, dass nur die x-Komponente $B_x$ des Magnetfelds als ungleich Null gegeben ist, wohingegen die y-Komponente $B_y$ und die z-Komponente $B_z$ im Wesentlichen Null sind.

Im Folgenden werden die zwei Vektoren $\vec{T}_x = [d_{peak\ width}, 0,0]^T$ und $\vec{T}_z = [0, 0, d_{peak\ width}]^T$ betrachtet, wobei $d_{peak\ width}$ ein positiver skalarer Entfernungswert, typischerweise im Bereich zwischen 0,01 und 0,1 m, ist. Durch die obigen Vektoren $\vec{T}_x$ und $\vec{T}_z$ werden vier Punkte $P_1, P_2, P_3, P_4$ in dem Arbeitsvolumen des Spulensystems wie folgt definiert:

$$P_1 \;=\; P - \vec{T}_z$$

$$P_2 \;=\; P + \vec{T}_x$$

$$P_3 \;=\; P + \vec{T}_z$$

$$P_4 \;=\; P - \vec{T}_x$$

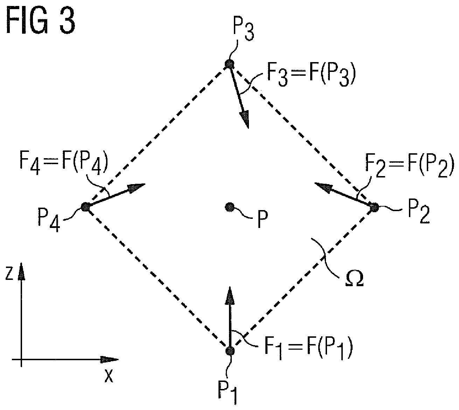

Diese Punkte sind beispielhaft in Fig. 3 verdeutlicht. Fig. 3 zeigt in der x-z-Ebene den spezifischen Punkt $P$, zu dem sich die Kapsel hinbewegen soll. Für diesen Punkt $P$ ist eine entsprechende Umgebung $\Omega$ gegeben, wobei der Rand der Umgebung ein Rechteck darstellt, welches in Fig. 3 gestrichelt wiedergegeben ist. Die entsprechenden Punkte $P_1$ bis $P_4$ liegen im Abstand $\vec{T}_x$ nach links bzw. rechts bzw. im Abstand $\vec{T}_z$ nach unten und oben versetzt von dem Punkt $P$. In der nachfolgenden Optimierung werden dabei die durch das Magnetfeld des Spulensystems erzeugten Kräfte $F_1 = F(P_1)$ am Punkt $P_1$, $F_2 = F(P_2)$ am Punkt $P_2$, $F_3 = F(P_3)$ am Punkt $P_3$ und $F_4 = F(P_4)$ am Punkt $P_4$ betrachtet, welche erfindungsgemäß immer in die Umgebung $\Omega$ hinein weisen müssen.

Insgesamt müssen die folgenden Randbedingungen zu der Erzeugung eines Magnetfeld-Maximums in der Umgebung des spezifischen Raumpunktes P gegeben sein:

1. Das Magnetfeld $\vec{B}$ an der spezifischen Position $P$ muss stark genug sein und korrekt ausgerichtet sein, so dass die erwünschte Orientierung der Kapsel erreicht wird. In einer ausreichend großen Umgebung um den Punkt $P$ muss deshalb das Magnetfeld $\vec{B}$ ungefähr die gleiche Richtung aufweisen. Diese Umgebung sollte wenigstens die obigen Positionen $P_1, P_2, P_3, P_4$ enthalten. Diese Bedingung ist dann erfüllt, wenn ein ausreichend starkes Magnetfeld an der spezifischen Raumposition $P$ gefordert wird.

2. Für die Punkte $P_1$, $P_2$, $P_3$, $P_4$ auf einer vorgegebenen konvexen Umgebung, welche gemäß Fig. 3 durch ein Rechteck repräsentiert wird, liegt die magnetische Kraft auf die Kapsel in jedem dieser Punkte innerhalb dieser konvexen Umgebung, d.h. die magnetische Kraft ist in die konvexe Umgebung hinein gerichtet. Auf diese Weise wird im Falle, dass keine externen Störungen vorliegen, sichergestellt, dass die Kapsel nach dem Eintritt in die konvexe Umgebung diese Umgebung niemals verlassen wird, da die Kräfte immer so gerichtet sind, dass die Kapsel in die Umgebung geschoben wird. Ferner wird sichergestellt, dass der Punkt, an dem die magnetische Kraft verschwindet, innerhalb dieser konvexen Umgebung in der Nähe des Punktes $P$ liegt. Demzufolge wird sich in Abwesenheit von externen Störungen die Kapsel immer hin zu dem spezifischen Punkt $P$ bewegen und dann auch dort verbleiben.

3. Der Absolutwert der Ströme in den einzelnen Spulen muss kleiner als jeweilige Maximalwerte sein. Ohne Beschränkung der Allgemeinheit wird in dem hier beschriebenen Ausführungsbeispiel angenommen, dass der maximale Strom für jede Spule gleich groß ist. Dieser Strom wird im Folgenden als $I_{max}$ bezeichnet.

Die obigen Bedingungen 1, 2 und 3 können als konvexes Optimierungsproblem beschrieben werden. In einer Variante wird die Optimierung als eine Maximierung der jeweiligen Ströme in den Einzelspulen beschrieben. Für einen vorgegebenen maximalen Strom $I_{max}$, ein minimales magnetisches Feld $B_{min}$ in x-Richtung und eine vorgegebene Konstante c (welche klein sein sollte und typischerweise kleiner als 0,01 ist) wird gemäß dem Optimierungsproblem nach Werten $I$, $\varepsilon$, $\delta$ gesucht, so dass gilt:

$$\max_{I,\delta,\varepsilon} \varepsilon$$

$$\varepsilon > 0,\ \delta > 0\ \ \delta < \varepsilon$$

$$B_x(P) > B_{min}$$

$$B_y(P) < cB_{min}$$

$$B_z(P) < cB_{min}$$

$$F_z(P_1) > \varepsilon$$

$$-\delta < F_x(P_1) < \delta$$

$$F_z(P_3) < -\varepsilon$$

$$-\delta < F_x(P_3) < \delta$$

$$-\delta < F_z(P_2) < \delta$$

$$F_x(P_2) < -\varepsilon$$

$$-\delta < F_z(P_4) < \delta$$

$$F_x(P_4) > \varepsilon$$

$$-I_{max} < I < I_{max}, \ komponentenweise$$

Dabei repräsentieren die Variablen $\varepsilon$ und $\delta$ Kraftwerte, welche gemäß der obigen Optimierung derart zu wählen sind, dass die Kraft, welche die Kapsel in das Zentrum des Rechtecks mit den Eckpunkten $P_1$ bis $P_4$ schiebt, maximiert wird, wobei entsprechende Beschränkungen in Bezug auf das magnetische Feld, die Kraftrichtung und die Ströme als Randbedingungen zu beachten sind. Die Randbedingung $0 < \delta < \varepsilon$ zusammen mit den Randbedingungen in Bezug auf die Komponenten der entsprechenden Kräfte $F_1$ bis $F_4$ an den Punkten $P_1$ bis $P_4$ stellen sicher, dass die magnetischen Kräfte immer in das durch die Punkte $P_1$ bis $P_4$ gebildete Rechteck hinein zeigen.

Das obige Optimierungsproblem ist ein lineares Programm, da die Kräfte und Felder lineare Kombinationen der Ströme sind. Die Lösung solcher Optimierungsprobleme ist hinlänglich aus dem Stand der Technik bekannt und es kann ein beliebiges Standardverfahren zur Lösung dieses Problems verwendet werden.

In einer zweiten Variante ist das Optimierungsproblem derart formuliert, dass ein Minimalwert des obigen Kraftwerts $\varepsilon$ vorgegeben ist und die euklidische Norm der Ströme minimiert wird. In diesem Fall wird für vorgegebenen Größen $\varepsilon$, $B_{min}$ und c nach Variablen $I$, $\delta$ gesucht, so dass gilt:

$$\min_{\delta,I} \left\| I \right\|^2$$

$$\delta > 0, \, \delta < \varepsilon$$

$$B_x(P) > B_{\min}$$

$$B_y(P) < cB_{\min}$$

$$B_z(P) < cB_{\min}$$

$$F_z(P_1) > \varepsilon$$

$$-\delta < F_x(P_1) < \delta$$

$$F_z(P_3) < -\varepsilon$$

$$-\delta < F_x(P_3) < \delta$$

$$-\delta < F_z(P_2) < \delta$$

$$F_x(P_2) < -\varepsilon$$

$$-\delta < F_z(P_4) < \delta$$

$$F_x(P_4) > \varepsilon$$

Gegebenenfalls kann auch bei diesem Optimierungsproblem die Randbedingung $-I_{\max} < I < I_{\max}$ berücksichtigt werden.

Im Gegensatz zum vorhergehenden Optimierungsproblem ist dieses Optimierungsproblem ein quadratisches Programm mit linearen Nebenbedingungen. Auch die Lösung solcher Optimierungsprobleme ist hinlänglich aus dem Stand der Technik bekannt, und es kann ein Standardverfahren für die Lösung verwendet werden. Die obigen Optimierungsprobleme können auch auf einfache Weise erweitert werden, indem unterschiedliche Gewichte für die Ströme berücksichtigt werden, so dass beispielsweise die Widerstandsverluste in dem Spulensystem minimiert werden.

Die im Vorangegangenen beschriebenen Ausführungsformen wurden am Beispiel einer Bewegung der Kapsel mit zwei translatorischen und drei rotatorischen Freiheitsgraden beschrieben. Gegebenenfalls kann die Erfindung auch für die Bewegung einer Kapsel mit weniger translatorischen bzw. rotatorischen Freiheitsgraden eingesetzt werden.

Die soeben erläuterten Varianten des Verfahrens weisen eine Reihe von Vorteilen auf. Insbesondere ist die mechanische Bewegung eines Patienten bzw. des Spulensystems während der medizinischen Untersuchung des Patienten nicht mehr erforderlich. Darüber hinaus ist es nicht mehr nötig, dass die Position der Kapsel in dem Arbeitsraum des Spulensystems gemessen werden muss, denn die Kapsel bewegt sich durch entsprechende Einstellung der Ströme unabhängig von ihrer aktuellen Position immer zu der Position des Magnetfeld-Maximums.

**Patentansprüche**

1. Spulensystem zur berührungslosen magnetischen Navigation eines magnetischen Körpers (1) in einem Arbeitsraum, wobei das Spulensystem eine Mehrzahl von Spulen und eine Stromsteuerung zur Steuerung der jeweiligen Ströme in der Mehrzahl von Spulen umfasst, wobei die Stromsteuerung derart ausgestaltet ist, dass zur Navigation des magnetischen Körpers (1) an eine variabel vorgebbare Position (P) in dem Arbeitsraum die Ströme in der Mehrzahl von Spulen eingestellt werden, **dadurch gekennzeichnet, dass** die Ströme derart eingestellt werden, dass die durch die Ströme generierten magnetischen Kräfte ($F_1$, $F_2$, $F_3$, $F_4$), welche auf den magnetischen Körper (1) an mehreren Positionen ($P_1$, $P_2$, $P_3$, $P_4$) am Rand einer konvexen Umgebung ($\Omega$) um die vorgebbare Position (P) wirken, in die Umgebung ($\Omega$) hinein weisen, wobei die Stromsteuerung eine Bewegung des magnetischen Körpers (1) mit zwei translatorischen Freiheitsgraden oder weniger berücksichtigt, und die zur Navigation des magnetischen Körpers (1) an die vorgebbare Position (P) einzustellenden Ströme die Lösung eines Optimierungsproblems mit der Randbedingung darstellen, dass die durch die Ströme generierten magnetischen Kräfte ($F_1$, $F_2$, $F_3$, $F_4$), welche auf den magnetischen Körper (1) an den mehreren Positionen ($P_1$, $P_2$, $P_3$, $P_4$) am Rand der konvexen Umgebung ($\Omega$) um die vorgebbare Position (P) wirken, in die Umgebung ($\Omega$) hinein weisen.

2. Spulensystem nach Anspruch 1, **dadurch gekennzeichnet, dass** die Umgebung ($\Omega$) derart ausgestaltet ist, dass der maximale Abstand des Rands der Umgebung ($\Omega$) zu der vorgebbaren Position (P) zwischen 0,005 und 0,1 Metern, insbesondere bei 0,01 Meter, liegt.

3. Spulensystem nach einem der vorhergehenden Ansprüche, **dadurch gekennzeichnet, dass** die Umgebung ($\Omega$) um die vorgebbare Position (P) ein Polygon und/oder ein Polyeder ist.

4. Spulensystem nach Anspruch 3, **dadurch gekennzeichnet, dass** die mehreren Positionen ($P_1$, $P_2$, $P_3$, $P_4$) an einem oder mehreren Eckpunkten des Polygons und/oder Polyeders, insbesondere an allen Eckpunkten, liegen.

5. Spulensystem nach einem der vorhergehenden Ansprüche, **dadurch gekennzeichnet, dass** die Stromsteuerung derart ausgestaltet ist, dass die Ströme im Betrieb des Spulensystems für die vorgebbare Position (P) durch die Stromsteuerung berechnet werden.

6. Spulensystem nach einem der vorhergehenden Ansprüche, **dadurch gekennzeichnet, dass** die einzustellenden Ströme für eine Vielzahl von vorgebbaren Positionen (P) in einem Speicher der Stromsteuerung hinterlegt sind.

7. Spulensystem nach einem der vorhergehenden Ansprüche, **dadurch gekennzeichnet, dass** das Optimierungsproblem ein lineares Programm und/oder ein quadratisches Programm ist.

8. Spulensystem nach einem der vorhergehenden Ansprüche, **dadurch gekennzeichnet, dass** das Optimierungsproblem die Minimierung des Betrags des Stromvektors umfassend die Ströme in der Mehrzahl von Spulen ist, wobei als weitere Randbedingung berücksichtigt wird, dass die durch die Ströme generierten magnetischen Kräfte ($F_1$, $F_2$, $F_3$, $F_4$), welche auf den magnetischen Körper (1) an den mehreren Positionen ($P_1$, $P_2$, $P_3$, $P_4$) am Rand der Umgebung ($\Omega$) um die vorgebbare Position (P) wirken, betragsmäßig jeweils einen vorbestimmten Wert überschreiten.

9. Spulensystem nach einem der vorhergehenden Ansprüche, **dadurch gekennzeichnet, dass** das Optimierungsproblem die betragsmäßige Maximierung der durch die Ströme generierten magnetischen Kräfte ($F_1$, $F_2$, $F_3$, $F_4$) ist, welche auf den magnetischen Körper (1) an den mehreren Positionen ($P_1$, $P_2$, $P_3$, $P_4$) am Rand der konvexen Umgebung ($\Omega$) um die vorgebbare Position (P) wirken, wobei als weitere Randbedingung berücksichtigt wird, dass die Ströme der Mehrzahl von Spulen betragsmäßig unterhalb eines jeweiligen Maximalwerts liegen.

10. Spulensystem nach einem der vorhergehenden Ansprüche, **dadurch gekennzeichnet, dass** bei der Lösung des Optimierungsproblems als zusätzliche Randbedingung berücksichtigt wird, dass das an der vorgebbaren Position (P) durch die Ströme erzeugte Magnetfeld im Wesentlichen in eine vorgegebene Richtung weist und einen Betrag aufweist, der einen vorgegebenen Wert überschreitet.

11. Spulensystem nach einem der vorhergehenden Ansprüche, **dadurch gekennzeichnet, dass** bei der Lösung des Optimierungsproblems die jeweiligen Ströme der Mehrzahl von Spulen unterschiedlich gewichtet werden.

**12.** Medizinisches Gerät, umfassend ein Spulensystem nach einem der vorhergehenden Ansprüche, welches derart ausgestaltet ist, dass ein Patient in den Arbeitsraum des Spulensystems positioniert werden kann und durch die Stromsteuerung des Spulensystems ein magnetischer Körper (1) in Form einer Sonde zur Untersuchung eines Organs des Patienten an vorgebbaren Positionen (P) innerhalb des Patientenkörpers navigiert werden kann.

**Claims**

**1.** Coil system for the contactless magnetic navigation of a magnetic body (1) in a work space, wherein the coil system comprises a plurality of coils and a current controller for controlling the respective currents in the plurality of coils, wherein in order to navigate the magnetic body (1) to a variably predeterminable position (P) in the work space, the current controller is designed such that the currents in the plurality of coils are adjusted, **characterised in that** the currents are adjusted such that the magnetic forces ($F_1$, $F_2$, $F_3$, $F_4$) generated by the currents and acting upon the magnetic body (1) at multiple positions ($P_1$, $P_2$, $P_3$, $P_4$) at the edge of a convex environment ($\Omega$) around the predeterminable position (P) are directed into the environment ($\Omega$), wherein the current controller takes account of a movement of the magnetic body (1) with two translational degrees of freedom or less, and the currents to be adjusted in order to navigate the magnetic body (1) to the predeterminable position (P) represent the solution to an optimisation problem with the boundary condition that the magnetic forces ($F_1$, $F_2$, $F_3$, $F_4$) generated by the currents and acting upon the magnetic body (1) at multiple positions ($P_1$, $P_2$, $P_3$, $P_4$) at the edge of the convex environment ($\Omega$) around the predeterminable position (P) are directed into the environment ($\Omega$).

**2.** Coil system according to claim 1, **characterised in that** the environment ($\Omega$) is designed in such a way that the maximal spacing of the edge of the environment ($\Omega$) from the predeterminable position (P) is between 0.005 and 0.1 meters, in particular 0.01 meters.

**3.** Coil system according to one of the preceding claims, **characterised in that** the environment ($\Omega$) around the predeterminable position (P) is a polygon and/or a polyhedron.

**4.** Coil system according to claim 3, **characterised in that** the multiple positions ($P_1$, $P_2$, $P_3$, $P_4$) are located at one or more corner(s) of the polygon and/or polyhedron, in particular at all corners.

**5.** Coil system according to one of the preceding claims, **characterised in that** the current controller is designed in such a way that the currents are calculated for the predeterminable position (P) by the current controller during operation of the coil system.

**6.** Coil system according to one of the preceding claims, **characterised in that** the currents to be adjusted for a large number of predeterminable positions (P) are stored in a memory of the current controller.

**7.** Coil system according to one of the preceding claims, **characterised in that** the optimization problem is a linear program and/or a quadratic program.

**8.** Coil system according to one of the preceding claims, **characterised in that** the optimisation problem is the minimisation of the value of the current vector comprising the currents in the plurality of coils, wherein as an additional boundary condition it is taken into account that the magnetic forces ($F_1$, $F_2$, $F_3$, $F_4$) generated by the currents and acting upon the magnetic body (1) at multiple positions ($P_1$, $P_2$, $P_3$, $P_4$) at the edge of the environment ($\Omega$) around the predeterminable position (P) exceed a predetermined value respectively.

**9.** Coil system according to one of the preceding claims, **characterised in that** the optimisation problem is the value of maximisation of the magnetic forces ($F_1$, $F_2$, $F_3$, $F_4$) generated by the currents and acting upon the magnetic body (1) at multiple positions ($P_1$, $P_2$, $P_3$, $P_4$) at the edge of a convex environment ($\Omega$) around the predeterminable position (P), wherein as an additional boundary condition it is taken into account that the value of the currents of the plurality of coils is below a respective maximum value.

**10.** Coil system according to one of the preceding claims, **characterised in that**, when solving the optimisation problem, it is taken into account as an additional boundary condition that the magnetic field generated by the currents at the predeterminable position (P) is substantially directed in a predetermined direction and has a value which exceeds a predetermined value.

**11.** Coil system according to one of the preceding claims, **characterised in that**, when solving the optimisation problem, the respective currents of the plurality of coils are weighted differently.

**12.** Medical device, comprising a coil system according to one of the preceding claims which is designed in such a way that a patient can be positioned in the work space of the coil system and a magnetic body (1) in the form of a probe for examining an organ in the patient can be navigated to predeterminable positions (P) inside the patient's body by the current controller of the coil system.

**Revendications**

**1.** Système de bobines pour la navigation magnétique sans contact d'un corps (1) magnétique dans un espace de travail, le système de bobines comprenant une pluralité de bobines et une commande de courant pour commander les courants respectifs dans la pluralité de bobines, la commande de courant étant conformée de manière à régler les courants dans la pluralité de bobines pour la navigation du corps (1) magnétique en une position (P) pouvant être donnée à l'avance de manière variable dans l'espace de travail,
**caractérisé en ce que**
les courants sont réglés de manière à ce que les forces ($F_1$, $F_2$, $F_3$, $F_4$) magnétiques produites par les courants, qui s'appliquent au corps (1) magnétique en plusieurs positions ($P_1$, $P_2$, $P_3$, $P_4$) au bord d'un voisinage ($\Omega$) convexe autour de la position (P) pouvant être donnée à l'avance, soient tournées vers le voisinage ($\Omega$), la commande de courant tenant compte d'un déplacement du corps (1) magnétique ayant deux degrés de liberté en translation ou moins et
les courants à régler pour la navigation du corps (1) magnétique en la position (P) pouvant être donnée à l'avance représentent la solution d'un problème d'optimisation avec la condition aux limites que les forces ($F_1$, $F_2$, $F_3$, $F_4$) magnétiques produites par les courants, qui s'appliquent au corps (1) magnétique en les plusieurs positions ($P_1$, $P_2$, $P_3$, $P_4$) au bord du voisinage ($\Omega$) convexe autour de la position (P) pouvant être donnée à l'avance, soient tournées vers le voisinage ($\Omega$).

**2.** Système de bobines suivant la revendication 1, **caractérisé en ce que** le voisinage ($\Omega$) est conformé de manière à ce que la distance maximum du bord du voisinage ($\Omega$) à la position (P) pouvant être donnée à l'avance soit comprise entre 0,005 et 0,01 mètre, en étant notamment de 0,01 mètre.

**3.** Système de bobines suivant l'une des revendications précédentes, **caractérisé en ce que** le voisinage ($\Omega$) autour de la position P pouvant être donnée à l'avance est un polygone et/ou un polyèdre.

**4.** Système de bobines suivant la revendication 3, **caractérisé en ce que** les plusieurs positions ($P_1$, $P_2$, $P_3$, $P_4$) sont en un ou en plusieurs sommets du polygone et/ou du polyèdre, notamment en tous les sommets.

**5.** Système de bobines suivant l'une des revendications précédentes, **caractérisé en ce que** la commande de courant est conformée de manière à ce que les courants soient, lorsque le système de bobines est en fonctionnement, calculés par la commande de courant pour la position (P) pouvant être donnée à l'avance.

**6.** Système de bobines suivant l'une des revendications précédentes, **caractérisé en ce que** les courants à régler sont, pour une pluralité de positions (P) pouvant être données à l'avance, mémorisés dans une mémoire de la commande de courant.

**7.** Système de bobines suivant l'une des revendications précédentes, **caractérisé en ce que** le problème d'optimisation est un programme linéaire et/ou un programme quadratique.

**8.** Système de bobines suivant l'une des revendications précédentes, **caractérisé en ce que** le problème d'optimisation est la minimisation de la valeur absolue du vecteur de courant, comprenant les courants dans la pluralité de bobines, dans lequel on tient compte, comme autre condition aux limites, que les forces ($F_1$, $F_2$, $F_3$, $F_4$) magnétiques produites par les courants, qui s'appliquent au corps (1) magnétique en les plusieurs positions ($P_1$, $P_2$, $P_3$, $P_4$) au bord du voisinage ($\Omega$) autour de la position (P) pouvant être donnée à l'avance, dépassent, en valeur absolue chacune, une valeur définie à l'avance.

**9.** Système de bobines suivant l'une des revendications précédentes, **caractérisé en ce que** le problème d'optimisation est la maximisation en valeur absolue des forces ($F_1$, $F_2$, $F_3$, $F_4$) magnétiques produites par les courants, qui s'appliquent au corps (1) magnétique en les plusieurs positions ($P_1$, $P_2$, $P_3$, $P_4$) au bord du voisinage ($\Omega$) convexe autour de la position (P) pouvant être donnée à l'avance, dans lequel on prend en compte, comme autre condition aux limites, que les courants de la pluralité de bobines sont, en valeur absolue, inférieurs à une valeur maximum respective.

**10.** Système de bobines suivant l'une des revendications précédentes, **caractérisé en ce que**, dans la solution du problème d'optimisation, on prend en compte, comme condition aux limites supplémentaires, que le champ magnétique produit par les courants en la position (P) pouvant être donnée à l'avance, est dirigé sensiblement dans une direction donnée à l'avance et a une valeur absolue qui dépasse une valeur donnée à l'avance.

**11.** Système de bobines suivant l'une des revendications précédentes, **caractérisé en ce que**, pour la solution du problème d'optimisation, les courants respectifs de la pluralité de bobines sont pondérés de manière différente.

**12.** Appareil de médecine, comprenant un système de bobines suivant l'une des revendications précédentes, qui est conformé de manière à ce qu'un patient puisse être mis en position dans l'espace de travail du système de bobines et de manière à ce que, par la commande de courant du système de bobines, un corps (1) magnétique sous la forme d'une sonde d'étude d'un organe du patient puisse être mis en navigation en des positions (P) pouvant être données à l'avance au sein du corps du patient.

FIG 1

FIG 2

FIG 3

$P_3$

$F_3 = F(P_3)$

$F_4 = F(P_4)$

$F_2 = F(P_2)$

$P_4$

$P$

$P_2$

$z$

$\Omega$

$x$

$F_1 = F(P_1)$

$P_1$

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2007077922 A1
- WO 2006014011 A1
- US 7019610 B2
- WO 2006092421 A1
- DE 10341092 A1